Europäisches Patentamt

(19) European Patent Office    (11) Numéro de publication: **0 133 830**

Office européen des brevets    **B1**

(12)    **FASCICULE DE BREVET EUROPEEN**

(45) Date de publication du fascicule du brevet:    (51) Int. Cl.⁴: **C 07 D 207/09, A 61 K 31/40**
07.01.88

(21) Numéro de dépôt: **84401564.4**

(22) Date de dépôt: **26.07.84**

(54) Application du N-(allyl 2-pyrrolidinylméthyl)2-méthoxy 4-amino 5-méthylsulfamoyl benzamide comme antiagrégant plaquettaire.

(30) Priorité: **10.08.83 FR 8313141**

(43) Date de publication de la demande:
**06.03.85 Bulletin 85/10**

(45) Mention de la délivrance du brevet:
**07.01.88 Bulletin 88/1**

(84) Etats contractants désignés:
**AT BE CH DE FR GB IT LI LU NL SE**

(56) Documents cités:
**EP - A - 0 047 207**

(73) Titulaire: **SOCIETE D'ETUDES SCIENTIFIQUES ET INDUSTRIELLES DE L'ILE-DE-FRANCE, 46, Boulevard de Latour-Maubourg, F-75340 Paris Cédex 07 (FR)**

(72) Inventeur: **Debay, André, 17, rue Letellier, F-75017 Paris (FR)**

(74) Mandataire: **Gallochat, Alain, SOCIETE D'ETUDES SCIENTIFIQUES ET INDUSTRIELLES DE L'ILE DE FRANCE 46, Boulevard de Latour Maubourg, F-75340 Paris Cédex 07 (FR)**

## Description

La présente invention concerne une nouvelle application du N-(1-allyl-2-pyrrolidinylméthyl) 2-méthoxy 4-amino 5-méthylsulfamoyl benzamide, ci-après appelé le «composé».

Les résultats d'études biochimiques in vitro ont montré que le composé inhibe l'agrégation plaquettaire provoquée par l'adénosine phosphate, et qu'il inhibe également la potentialisation par la clonidine de l'agrégation due à l'adénosine phosphate.

Des observations attentives de ce mécanisme chez le lapin ont montré que le composé ne modifie pas le captage de la sérotonine par les plaquettes, mais inhibe sa libération, induite par l'adrénaline à partir de ces mêmes plaquettes.

Enfin, le composé s'oppose à l'action agrégante plaquettaire du collagène, prolongeant la phase de latence. Il inhibe la libération de la sérotonine par les plaquettes sous l'effet du collagène, se montrant dans cet effet 13 fois plus actif que l'aspirine.

Des essais ont été faits de même in vitro sur des plaquettes humaines; dans ces conditions il s'est révélé que le composé inhibe la seconde phase de l'agrégation induite par la thrombine, et l'agrégation provoquée par le collagène, l'adrénaline et la sérotonine.

De plus, il inhibe la libération de la sérotonine, induite par la thrombine ou le collagène. Il inhibe également la réaction de libération du matériel intraplaquettaire provoquée par l'adrénaline, comme le prouve l'inhibition de la seconde phase de l'agrégation habituellement associée à cette libération.

Des essais in vivo chez l'animal ont confirmé l'effet antiagrégant du composé, celui-ci modifiant les conditions de formation d'un thrombus vasculaire provoqué.

Des anomalies de la fonction plaquettaire ont été rapportées au cours de divers troubles thrombo-emboliques, tels que ischémie cardiaque, atteinte cérébro-vasculaire, thrombose veineuse idiopathique, de même que dans des cas d'hyperlipoprotéinémie, d'hypertension, de diabète, ou chez les gros fumeurs, tous symptômes bien connus d'aggravation du risque cardiovasculaire.

Bien que dans ces conditions le rôle pathogénétique du dysfonctionnement plaquettaire n'ait pas été définitivement établi, des médicaments variés, supposés agir en interférant avec la fonction plaquettaire ont été utilisés largement. Citons par exemple l'aspirine, le dipyridamole, la sulfinpyrazone. Les études pharmacologiques ont démontré chez l'animal, que le composé n'agissait que très faiblement sur les récepteurs dopaminergiques centraux; en particulier, il faut des doses élevées pour antagoniser les effets classiques à l'apomorphine, comprenant le redressement et les stéréotypies chez le rongeur. On peut donc dire que les effets neurologiques indésirables sont très limités avec ce médicament.

Les résultats pharmacologiques suivants illustrent l'activité du composé, sur l'agrégation plaquettaire, la potentialisation de cette agrégation, le captage de la sérotonine (dont le nom chimique est 5-hydroxytryptamine) par les plaquettes et la réaction de libération de ces plaquettes. Cette étude a été réalisée sur le lapin:

- On observe un effet inhibiteur sur l'agrégation des plaquettes induite par l'adénosine phosphate.
- Le composé inhibe la potentialisation (induite par la 5-hydroxytryptamine) de l'agrégation des plaquettes provoquée par l'adénosine phosphate, à la $CI_{50}$ (concentration inhibitrice 50) de 19 μM. Il est 63 fois moins puissant que le méthysergide comme antagoniste de la 5-hydroxytryptamine.
- La composé inhibe la potentialisation (induite par l'adrénaline) de l'agrégation plaquettaire provoquée par l'adénosine phosphate, à la $CI_{50}$ de 10 μM. Il est 20 fois moins puissant que la phentolamine comme antagoniste de l'adrénaline.
- L'effet anti-adrénergique du composé ($CI_{50}$ = 29,5 μM) intrevient, au moins en partie, au moyen du blocage des récepteurs alpha-2. Ceci est démontré par l'inhibition, obtenue par le composé de l'invention, de la potentialisation (induite par la clonidine) de l'agrégation plaquettaire provoquée par l'adénosine phosphate, ce qui constitue un effet mettant en jeu les récepteurs alpha-2. Le composé est deux fois plus actif que la miansérine comme antagoniste alpha-2.
- Le composé n'affecte pas de manière significative le captage de la 5-hydroxytryptamine marquée au carbone 14, par les plaquettes du lapin ($CI_{50} > 100$ μM).
- Le composé antagonise l'action du collagène sur les plaquettes. Ceci est démontré par:

a) la prolongation de la phase de latence typique qui précède l'agrégation des plaquettes, induite par la collagène,

b) l'inhibition de l'agrégation induite par le collagène,

c) l'inhibtion de la réaction de libération par les plaquettes (libération de la 5-hydroxytryptamine marquée au carbone 14, induite par le collagène), avec une $CI_{50}$ de 1,8 μM. Le composé selon l'invention est 13 fois plus puissant que l'aspirine, bien connue pour inhiber la réaction de libération des plaquettes.

- Le composé inhibe la libération de la 5-hydroxytryptamine marquée au carbone 14, (induite par l'adrénaline) des plaquettes du lapin, à une $CI_{50}$ de 100 μM).

Tous ces résultats concordant, l'étude suivante a été réalisée sur des plaquettes humaines in vitro, avant de procéder aux applications thérapeutiques. Elle porte sur les effets du composé selon l'invention, à l'égard de l'agrégation induite par la thrombine, le collagène, l'adrénaline, la 5-hydroxytryptamine, et à l'égard de la libération de la 5-hydroxytryptamine marquée au carbone 14, par la thrombine, l'adrénaline et le collagène. Cette série d'études a conduit aux résultats suivants:
– Le composé n'influence pas l'agrégation primaire des plaquettes, mais inhibe la seconde phase de l'agrégation induite par la thrombine (3,3 unités/ml PRP).
– Le composé inhibe l'agrégation des plaquettes, induite par le collagène.
– Le composé inhibe à la fois la première et la seconde phase de l'agrégation des plaquettes, induite par l'adrénaline.
– Le composé inhibe l'agrégation des plaquettes induite par la 5-hydroxytryptamine.
– Le composé n'influence pas le captage de la 5-hydroxytryptamine marquée au carbone 14, par les plaquettes.
– Le composé inhibe la libération de la 5-hydroxytryptamine marquée au carbone 14, induite par la thrombine, à partir des plaquettes.
– Le composé inhibe la libération de la 5-hydroxytryptamine marquée, induite par le collagène, à partir des plaquettes.
– Le composé inhibe la réaction de libération des plaquettes, induite par l'adrénaline, comme en témoigne l'inhibition de la seconde phase de l'agrégation habituellement associée à la libération du matériel intraplaquettaire.

Le composé selon l'invention, ayant confirmé sur les plaquettes humaines les données obtenues au cours des études sur l'animal, peut être proposé en thérapeutique, comme médicament antiagrégant, et selon le but poursuivi, à des posologies variant de 15 mg à 150 mg par jour par voie buccale. Le composé est généralement utilisé, associé à un excipient inerte par rapport au principe actif, sous différentes formes galéniques usuelles. Bien que la forme injectable ou rectale soit réalisable, la forme orale est préférée: on utilisera donc plutôt des comprimés, gélules, capsules, sirops ou soluté buvable, facilitant l'étalement du traitement au cours de la journée.

En conclusion, il est proposé le N-(1-allyl-2-pyrrolidinylméthyl) 2-méthoxy 4-amino 5-méthylsulfamoyl benzamide comme nouveau médicament à effet antiagrégant plaquettaire.

**Revendication**

1. Utilisation du N-(1-allyl 2-pyrrolidinylméthyl) 2-méthoxy 4-amino 5-méthylsulfamoylbenzamide et de ses sels pharmacologiquement acceptables pour la préparation d'un médicament servant d'antiagrégant plaquettaire.

**Claim**

Use of N-(1-allyl 2-pyrrolidinylmethyl) 2-methoxy 4-amino 5-methylsulphamoyl benzamide and pharmacologically acceptable salts thereof for the preparation of a platelet anti-aggregating medicament.

**Patentanspruch**

1. Verwendung von N-(1-Allyl-2-pyrrolidinylmethyl)-2-methoxy-4-amino-5-methylsulfamoylbenzamid und seiner pharmakologisch verträglichen Salze für die Herstellung eines als Blutplättchen-Antiaggregationsmittel dienenden Medikaments.